# EUROPÄISCHE PATENTSCHRIFT

(11) **EP 0 550 791 B1**
(45) Veröffentlichungstag und Bekanntmachung des Hinweises auf die Patenterteilung: **21.08.1996**
(21) Anmeldenummer: 92111244.7
(22) Anmeldetag: 02.07.1992
(51) Int. Cl.: A61F 9/00

(54) **Vorrichtung zur Aufrechterhaltung des natürlichen Augen-Innendruckes**
Device to keep intraocular pressure at its natural level
Dispositif pour maintenir le niveau naturel de la pression oculaire interne

(30) Priorität: 10.01.1992 CH 62/92
(43) Veröffentlichungstag der Anmeldung: 14.07.1993
(73) Patentinhaber: GRIESHABER & CO. AG SCHAFFHAUSEN, CH-8203 Schaffhausen (CH)
(72) Erfinder: Stegmann, Robert, Prof.M.D., Pretoria, 0181 (ZA); Grieshaber, Hans Rudolf, CH-8200 Schaffhausen (CH)
(74) Vertreter: Althoff, Gerhard

(56) Entgegenhaltungen:
- EP-A- 0 348 146
- FR-A- 2 466 994
- US-A- 4 586 921
- US-A- 4 759 746
- US-A- 4 846 172
- Section PQ, Week 42, 25. November 1981 Derwent Publications Ltd., London, GB; Class P, AN K9310 & SU-A-799 746 ( OMSK MED. INST. )
- Section PQ, Week 9232, Derwent Publications Ltd., London, GB; Class P, AN 92- 266123 & SU-A-1 644 954 ( EYE DISEASE RES. INST. )
- Section PQ, Week 9109, Derwent Publications Ltd., London, GB; Class P, AN 91- 063767 & SU-A-1 572 615 ( CHEREDNICHENKO V.I. )

## Beschreibung

Die Erfindung bezieht sich auf eine Vorrichtung zur Wiederherstellung des vom Zu- und Abfluss des Kammerwassers abhängigen natürlichen Innendruckes im Auge eines Lebewesens, bestehend aus einem Injektionsgerät und einer damit in Verbindung stehenden röhrchenförmigen Sonde, welche durch einen Einschnitt in den etwa kreisringförmigen Schlemmschen Kanal einführbar ist.

Für die einwandfreie Funktionsfähigkeit des Auges ist es unter anderem erforderlich, dass der Druck des sich kontinuierlich erneuernden und zwischen der Hinterkammer sowie der Vorderkammer zirkulierenden Kammerwassers derart ausgeglichen wird, dass der Ab- und Zufluss des Kammerwassers in gleichem Verhältnis steht und der Abfluss desselben über das dem Schlemmschen Kanal vorgelagerte Trabekulargewebe gewährleistet ist.

Störungen des Kammerwasserabflusses können beispielsweise bei einem den Zugang zum Schlemmschen Kanal spaltförmig verengenden Kammerwinkel oder aber auch bei krankhaften und die Durchlässigkeit des Kammerwassers verhindernden Veränderungen des Trabekulargewebes auftreten. Ist der Abfluss des Kammerwassers geringer als dessen Zufluss, so steigt der Druck im Inneren des Auges, wodurch die unter dem Begriff "Glaukom" bekannte und oftmals zur Blindheit führende Sehstörung entsteht.

Aus der SU-A 799,746 ist zum Spülen des Schlemmschen Kanals ein Verfahren bekannt, bei welchem etwa ein segmentförmiger Abschnitt der Kanalwand durch einen mikrochirurgischen Einschnitt geöffnet und anschliessend eine entsprechend dem Lumen des Kanals ausgebildete Kanüle etwa 2 mm bis 3 mm in den Kanal eingeführt und in einer nächsten Phase der geöffnete Kanalbereich mit einer geeigneten physiologischen Lösung gespült wird. Mit dieser Spülmethode kann der Abfluss des Kammerwassers in dem relativ kleinen, geöffneten Bereich über die natürlichen Drainagen im Trabekulargewebe erreicht werden. Ein Öffnen der natürlichen und bei krankhaftem Trabekulargewebe verstopften Drainagen ist mit der bekannten Spülmethode nicht möglich.

Der Erfindung liegt die Aufgabe zugrunde, eine Vorrichtung zum Injizieren eines Mediums zu schaffen, mittels welchem das dem Schlemmschen Kanal vorgelagerte und verstopfte und/oder zugewachsene Venennetz des Trabekulargewebes geöffnet und dadurch der den Druckausgleich bewirkende, natürliche Abfluss des sich ständig erneuernden Kammerwassers gewährleistet ist.

Diese Aufgabe wird dadurch gelöst, dass die Sonde kreisbogenförmig ausgebildet und jeweils an der Bogeninnenseite und/oder an dem einen im Schlemmschen Kanal angeordneten Ende mit mindestens einer Austrittsöffnung versehen ist über welche von dem an dem anderen Ende der Sonde angeordneten Injektionsgerät ein Medium injizierbar ist, mittels welchem eine hydraulische Dehnung des Schlemmschen Kanals sowie ein zwangsläufiges Aufplatzen des vorgelagerten Trabekulargewebes erreichbar ist und dadurch eine Verbindung zwischen dem Schlemmschen Kanal und dem kanalförmigen Venennetz des Trabekulargewebes herstellbar ist.

Mit der im wesentlichen als kreisbogenförmiges Segmentteil ausgebildeten Sonde sowie der Anordnung der Austrittsöffnungen sind diese beim Einführen in den Schlemmschen Kanal zwangsläufig dem vorgelagerten Trabekulargewebe zugewandt, so dass beim Einpressen des Mediums ein Aufplatzen des Gewebes an mehreren Stellen erreicht wird und dadurch der Abfluss des Kammerwassers wieder gewährleistet ist.

Die Verwendung eines hochviskosen Mediums, insbesondere einer chemisch modifizierten Hyaluronsäure sowie Abwandlungen derselben haben den Vorteil, dass die beim traumatischen Aufplatzen entstehenden Öffnungen im Trabekulargewebe gleichzeitig benetzt und dadurch Blutungen verhindert werden, so dass eine Neubildung des Gewebes sowie ein Verschliessen der Öffnungen weitgehend ausgeschlossen ist.

Weitere Merkmale der Erfindung ergeben sich aus der folgenden Beschreibung in Verbindung mit den in der Zeichnung dargestellten Ausführungsbeispielen.

Die Erfindung wird nachstehend anhand der Zeichnung beschrieben. Es zeigt:
- Fig. 1: ein schematisch und im Schnitt dargestelltes Auge eines Lebewesens mit dem vorderen und hinteren Augenabschnitt;
- Fig. 2: den in grösserem Massstab und im Schnitt dargestellten vorderen Augenabschnitt;
- Fig. 3: ein in Ansicht mit lamellar eingeschnitten und aufgeklappter Lederhaut sowie teilweise freigelegtem Schlemmschen Kanal dargestelltes Teilstück des Auges;
- Fig. 4: ein in Ansicht und in grösserem Massstab dargestelltes Teilstück des Auges mit einer in den Schlemmschen Kanal eingeführten Sonde,
- Fig. 5: ein erstes Ausführungsbeispiel und in Schnittansicht dargestelltes Teilstück der Sonde,
- Fig. 6: ein zweites Ausführungsbeispiel und in Schnittansicht dargestelltes Teilstück der Sonde,
- Fig. 7: ein drittes Ausführungsbeispiel und in Ansicht dargestelltes Teilstück der Sonde, und
- Fig. 8: zwei für den kreisringförmigen Schlemmschen Kanal spiegelbildlich ausgebildete Sonden mit jeweils daran angeordnetem Stutzen für den Anschluss an ein Injektionsgerät.

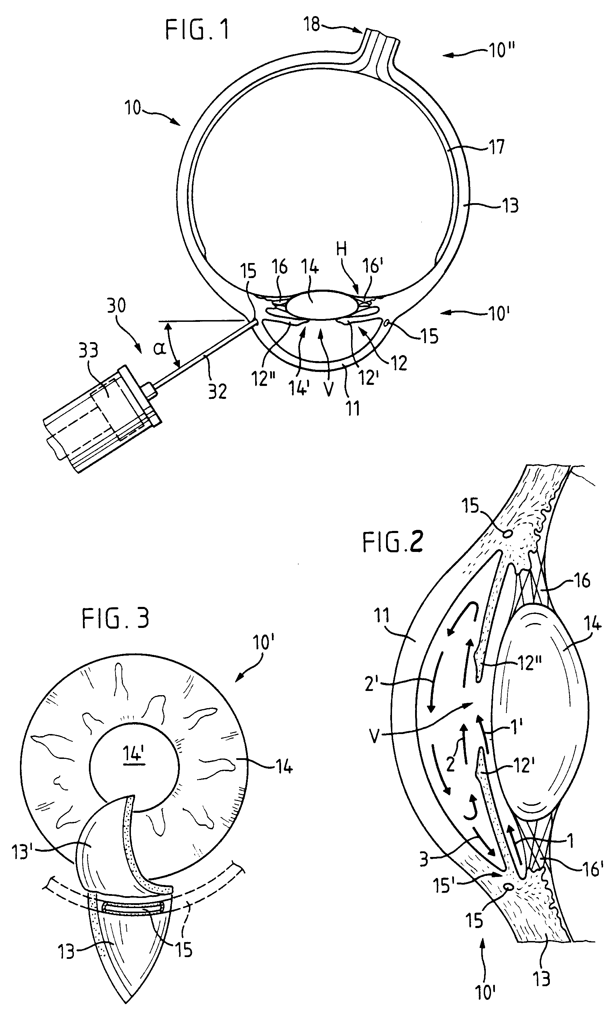

Fig.1 zeigt ein im Schnitt dargestelltes und in der Gesamtheit mit 10 bezeichnetes Auge, bei welchem mit 10'' der hintere Augenabschnitt und mit 10' der vordere Augenabschnitt bezeichnet ist.

Im vorderen Augenabschnitt 10' des Auges 10 erkennt man in Fig.1 weiterhin die Hornhaut 11 (Cornea), die Regenbogenhaut 12 (Iris) mit den beiden Bereichen 12' und 12'', die Lederhaut 13 (Sklera), die Linse 14 (Okular) mit der Pupille 14', die Strahlenbänder 16,16' sowie den Schlemmschen Kanal 15 (Sinus venosus sclerae). Der etwa in der Winkelspitze des Übergangs von der Hornhaut 11 zur Lederhaut 13 liegende, kreisringförmige Schlemmsche Kanal 15 verläuft weitgehend parallel zum Rand der Hornhaut 11. Weiterhin erkennt man in Fig.1 die Netzhaut 17 (Retina) sowie den in der Gesamtheit mit 18 bezeichneten Sehnerv (Optikus).

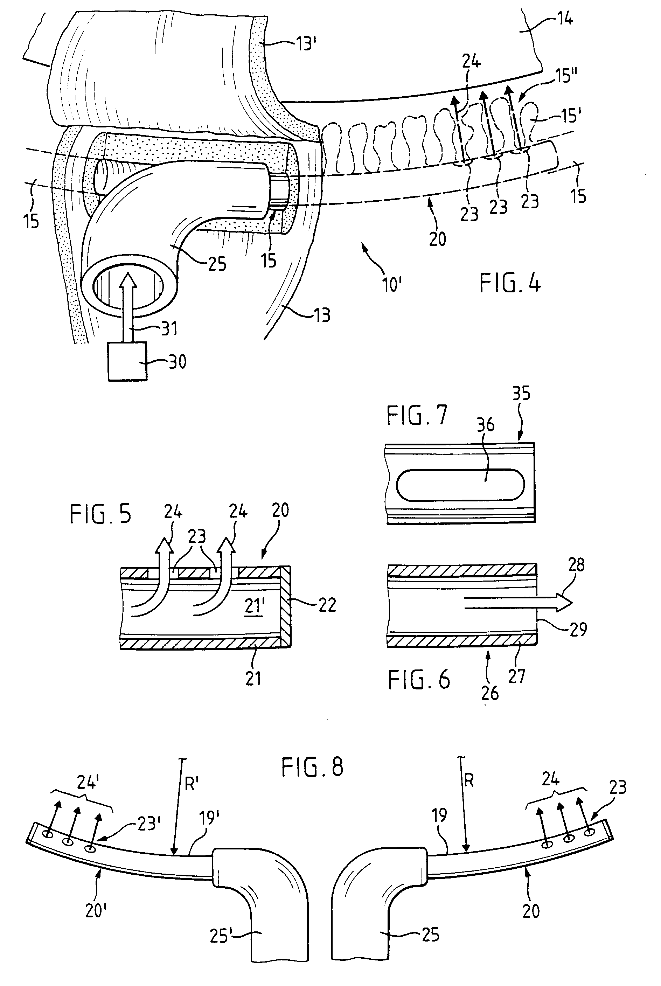

Bei einem gesunden Auge erfolgt über den Schlemmschen Kanal 15 sowie über das in Fig.4 dargestellte, vorgelagerte und mit natürlichen Öffnungen 15'' versehene Trabekulargewebe 15' (Trabeculum corneosclerae) der Abfluss des sich ständig erneuernden und zwischen der Hinterkammer H und der Vorderkammer V (Fig.1) zirkulierenden Kammerwassers. Der Ab- und Zufluss des Kammerwassers (nicht dargestellt) steht bei einem gesunden Auge in gleichem Verhältnis. Zur Vereinfachung wird nachstehend der Schlemmsche Kanal 15 als Kanal 15 und das demselben vorgelagerte Trabekulargewebe 15' als Gewebe 15' bezeichnet.

Bei einem kranken Auge kann sich der Kanal 15 und/oder das vorgelagerte Gewebe 15' mit den natürlichen Öffnungen 15'' sowie das natürliche, nicht dargestellte kanalförmige Venennetz derart verschliessen, dass der Abfluss des Kammerwassers geringer als der Zufluss ist. Dabei steigt der Druck im Inneren des Auges 10 derart an, dass der Sehnerv 18 eingeklemmt wird. Diese unter dem Begriff "Glaukom" bekannte Sehstörung führt oftmals zur Blindheit des betroffenen Auges oder beider Augen.

Zur Behandlung des einzelnen kranken Auges 10 wird mittels eines Injektionsgerätes 30 (Fig.1) ein hochviskoses Medium in den Kanal 15 injiziert bis das vorgelagerte Gewebe 15', wie in Fig.4 schematisch dargestellt, durch die hydraulische Dehnung aufplatzt. Das Injektionsgerät 30 umfasst, wie in Fig.1 schematisch dargestellt, mindestens eine Spritze 33 sowie eine entsprechend in den Kanal 15 eingeführte und in Fig.1 nicht näher dargestellte Sonde, welche über eine Zuführleitung 32 mit der Spritze 33 in Verbindung steht. Die Spritze 33 ist beispielsweise eine an sich bekannte, auswechselbare Kolbenspritze, welche zum Injizieren des hochviskosen Mediums entweder manuell oder aber mit nicht dargestellten Mitteln elektronisch gesteuert betätigbar ist.

Beim Injizieren des hochviskosen Mediums ist das Injektionsgerät 30 mit der Zuführleitung 32, wie in Fig.1 dargestellt, unter einem in der Grössenordnung von 45° liegenden Winkel α in bezug auf den Kanal 15 angeordnet.

In Fig.2 ist in grösserem Massstab und im Schnitt der vordere Augenabschnitt 10' des Auges 10 dargestellt, und man erkennt die Hornhaut 11, die beiden Bereiche 12' und 12'' der Regenbogenhaut 12, die Lederhaut 13, die Linse 14 mit den Strahlenbändern 16,16' sowie den Kanal 15. Das schematisch mit den Pfeilen 1,1' und 2,2' im Bereich der Vorderkammer V zirkulierend dargestellte Kammerwasser wird gemäss Pfeilrichtung 3 dem Kanal 15 zugeführt und von diesem in nicht näher dargestellter Weise über das vorgelagerte Gewebe 15' (Fig.4) und über das nicht dargestellte kanalförmige Venennetz abgeführt.

Fig.3 zeigt den in schematischer Ansicht dargestellten vorderen Augenabschnitt 10', und man erkennt die Linse 14 mit der Pupille 14' sowie die teilweise dargestellte Lederhaut 13. Zur Freilegung und Öffnung eines Abschnitts des die Linse 14 zirkulär umgebenden Kanals 15 wird die Lederhaut 13 operativ und lamellar eingeschnitten und der äussere Teil als lappenförmiges Teilstück 13' entsprechend aufgeklappt. Das aufgeklappte Teilstück 13' der Lederhaut 13 wird dabei mit nicht dargestellten Mitteln gehalten.

In Fig.4 ist in grösserem Massstab ein Teilstück des vorderen Augenabschnitts 10' mit dem teilweise freigelegten Kanal 15 und dem aufgeklappten Teilstück 13' der Lederhaut 13 dargestellt. In den Kanal 15 ist eine röhrchenförmige Sonde 20 eingeführt, welche auf der dem vorgelagerten Gewebe 15' zugewandten Oberfläche mit im Abstand zueinander angeordneten Öffnungen 23 versehen ist. Die Sonde 20 hat vorzugsweise nur im Endbereich zwei oder drei in Reihe angeordnete Öffnungen 23. An dem einen Ende der Sonde 20 ist ein bogenförmiges Anschlussstück 25 angeordnet, welches in nicht näher dargestellter Weise kupplungsartig mit der Zuführleitung 32 des Injektionsgerätes 30 (Fig.1) verbunden wird. Von dem Injektionsgerät 30 wird gemäss Pfeilrichtung 31 über die Sonde 20 das hochviskose Medium in den Kanal 15 injiziert.

Beim Injizieren des Mediums wird der Kanal 15, wie in Fig.4 schematisch dargestellt, an der geringer abgestützten und der Vorderkammer V (Fig.1,2) zugewandten Seite hydraulisch derart gedehnt, dass das dem Kanal 15 vorgelagerte Gewebe 15' anschliessend an den schwächsten Stellen in Pfeilrichtung 24 aufplatzt und dabei jeweils eine Öffnung 15'' im Gewebe 15' bildet. Die neu gebildeten Öffnungen 15'' sind in bezug auf die in der Sonde 20 vorgesehenen Öffnung 23 korrespondierend angeordnet.

In Fig.4 wird mittels der Sonde 20 das eine durch den Einschnitt freigelegte und geöffnete Teilstück (rechts) des Kanals 15 behandelt. Mittels einer spiegelbildlich ausgebildeten und in das andere Teilstück (links) des Kanals 15 eingeführten Sonde 20' (Fig.8) wird anschliessend das andere Teilstück des Kanals 15 behandelt. Die Behandlung der beiden Teilstücke des Kanals 15 erfolgt vorzugsweise nacheinander, wobei jeweils die gegenüberliegende Sonde aus dem Kanal 15 entfernt wird.

Fig.5 zeigt als erstes Ausführungsbeispiel ein Teilstück der in Schnittansicht sowie in wesentlich grösserem Massstab dargestellten Sonde 20. Die röhrchenförmig ausgebildete Sonde 20 hat einen dem Innendurchmesser des Kanals 15 angepassten Aussendurchmesser, welcher in der Grössenordnung von etwa 0,15 mm liegt. Die Sonde 20 kann an dem einen Ende, welches in den Kanal 15 (Fig.4) eingeführt wird, mit einer Stirnplatte 22 oder dergleichen verschlossen sein. Am Ende der in den Kanal 15 eingeführten Sonde 20 sind Öffnungen 23 vorgesehen, welche die Wand 21 durchdringend mit dem Innenraum 21' der Sonde 20 in Verbindung stehen.

Vorzugsweise sind im letzten Drittel der Sonde 20 zwei bis vier mit dem Innenraum 21' in Verbindung stehende Öffnungen 23 angeordnet, über welche das hochviskose Medium, wie in Fig.4 dargestellt, in Pfeilrichtung 24 durch das Gewebe 15' entweichen kann. Die in Reihe und im Abstand zueinander angeordneten Öffnungen 23 können dabei gleich gross oder aber unterschiedlich gross dimensioniert sein.

Bei dem in Fig.6 dargestellten zweiten Ausführungsbeispiel ist eine röhrchenförmige Sonde 26 mit einer einzigen, stirnseitigen Öffnung 29 versehen, über welche das hochviskose Medium in Pfeilrichtung 28 in den Kanal 15 (Fig.4) eingebracht wird. In der Wand 27 sind bei diesem Ausführungsbeispiel keine weiteren Öffnungen vorgesehen.

Fig.7 zeigt als drittes Ausführungsbeispiel ein in Ansicht dargestelltes Teilstück einer Sonde 35, welche mit einer als Langloch ausgebildeten Öffnung 36 versehen ist, über welche das hochviskose Medium in den Kanal 15 (Fig.4) eingebracht (injiziert) wird. Bei einer nicht näher dargestellten Variante kann die Öffnung 36 auch als ein bis zum Ende reichender Schlitz ausgebildet sein.

An dieser Stelle sei darauf hingewiesen, dass die jeweilige Sonde 20,26 oder 35 im Endbereich mit unterschiedlich ausgebildeten Öffnungen versehen sein kann. Beispielsweise kann die in Fig.5 dargestellte und mit den Öffnungen 23 versehene Sonde 20 stirnseitig, analog wie die Sonde 26 gemäss Fig.6, mit einer Öffnung 29 versehen sein.

Fig.8 zeigt zwei in Ansicht dargestellte und spiegelbildlich ausgebildete Sonden 20 und 20', welche für die Behandlung getrennt in den Kanal 15 (Fig.4) eingeführt werden, d.h., dass beispielsweise erst die eine Sonde 20 eingeführt und nach der Behandlung entfernt und anschliessend die andere Sonde 20' in den Kanal 15 eingeführt wird.

Die einzelne Sonde 20 oder 20' ist bogenförmig ausgebildet und hat einen dem im wesentlichen kreisringförmigen Kanal 15 angepassten Radius R oder R'. Der Radius R oder R' liegt in der Grössenordnung von etwa 12 mm bis 14 mm. Weiterhin erkennt man in Fig.7 die an dem einen Ende der Sonde 20 oder 20' im Endbereich an der Bogeninnenseite 19 oder 19' angeordneten Öffnungen 23 oder 23' sowie den jeweils am anderen Ende angeordneten Stutzen 25 oder 25' zum Anschliessen an das hier nicht dargestellte Injektionsgerät 30 (Fig.4). Aus den Öffnungen 23,23' wird etwa gemäss Pfeilrichtung 24,24' das Medium in Richtung des Gewebes 15' (Fig.4) injiziert.

Die Sonde 20,20',26 oder 35 ist vorzugsweise aus rostfreiem Stahl hergestellt, wobei ein geeigneter Kunststoff ebenfalls Anwendung finden kann.

Bei dem zu injizierenden Medium handelt es sich um ein hochviskoses Gel, mittels welchem die Oberflächen der traumatisch hergestellten Öffnungen 15'' benetzt werden, so dass die drainagenartige Funktion des Kanals 15 auch nach Auflösung des Gels auf längere Zeit gewährleistet ist.

Das einzuspritzende Medium muss physiologisch und ophthalmologisch verträglich sein und darf im Kanal 15 sowie im Gewebe 15' keine unerwünschten Nebeneffekte verursachen. Mit dem Medium soll einerseits die beim traumatischen Öffnen des Kanals 15 auftretende Blutung vermieden werden, und andererseits muss die Benetzung der Öffnungen 15'' solange bestehenbleiben, bis keine lokale Gewebeverbindung (Zellen- und Narbenbildung) mehr möglich ist.

Ein bevorzugtes, physiologisch und ophthalmologisch verträgliches Medium ist beispielsweise aus der US-A 4 141 973 sowie der US-A 4 713 448 bekannt. Bei dem bekannten Medium handelt es sich um eine hochviskose, wässrige Lösung, welche mit sogenannten Pufferzusätzen, wie Phosphaten und/oder Salzen, angereichert ist.

Das Medium ist beispielsweise eine wässrige Lösung des Natriumsalzes von Hyaluronsäure, welches ein Glykosaminoglykan mit hohem Molekulargewicht bildet, wobei das Glykosaminoglykan eine chemisch modifizierte Hyaluronsäure ist. Das Molekulargewicht liegt vorzugsweise in der Grössenordnung von 3,2 x 10⁶ bis 5 x 10⁶.

An dieser Stelle sei darauf hingewiesen, dass auch wässrige Lösungen mit geringerer Viskosität Anwendung finden können. Hierbei ist es jedoch erforderlich, dass das Medium ophthalmologisch verträgliche Antikoagulations-Substanzen, wie beispielsweise Epsilon-Aminokaproische Säure, enthält.

Es können auch weitere, ophthalmologisch vergleichbare und auf Hyaluronsäure basierende Medien, wie beispielsweise Hydroxypropylmethylzellulose, Polyacrylamide, Mucopolysaccharide, Chondroitinsulfat oder andere Typen von Polysacchariden, verwendet werden. Weiterhin können auch Mischungen von Substanzen, wie beispielsweise Hyaluronsäure mit Chondroitinsulfat oder Hyaluronsäure mit Dextran, verwendet werden.

## Patentansprüche

1. Vorrichtung zur Wiederherstellung des vom Zu- und Abfluss des Kammerwassers abhängigen natürlichen Innendruckes im Auge eines Lebewesens, bestehend aus einem Injektionsgerät und einer damit in Verbindung stehenden röhrchenförmigen Sonde, welche durch einen Einschnitt in den etwa kreisringförmigen Schlemmschen Kanal einführbar ist, **dadurch gekennzeichnet**, dass die Sonde (20;20';26;35) kreisbogenförmig ausgebildet und jeweils an der Bogeninnenseite (19;19') und/oder an dem einen im Schlemmschen Kanal (15) angeordneten Ende mit mindestens einer Austrittsöffnung (23;23';29;36) versehen ist über welche von dem an dem anderen Ende der Sonde (20;20'; 26;35) angeordneten Injektionsgerät (30) ein Medium injizierbar ist, mittels welchem eine hydraulische Dehnung des Schlemmschen Kanals (15) sowie ein zwangsläufiges Aufplatzen des vorgelagerten Trabekulargewebes (15') erreichbar ist und dadurch eine Verbindung zwischen dem Schlemmschen Kanal (15) mit dem kanalförmigen Venennetz des Trabekulargewebes (15') herstellbar ist.

2. Vorrichtung nach Anspruch 1, **dadurch gekennzeichnet,** dass die aus einem Röhrchen hergestellte Sonde (20;20';26;35) als kreisbogenförmiges Segmentteil ausgebildet ist und jeweils einen etwa dem Schlemmschen Kanal (15) angepassten und in der Grössenordnung von 12 mm bis 14 mm liegenden Radius (R,R') aufweist.

3. Vorrichtung nach den Ansprüchen 1 und 2, **dadurch gekennzeichnet,** dass die Sonde (20;20';35) an der Bogeninnenseite (19;19') jeweils mit mehreren im Abstand zueinander angeordneten Austrittsöffnungen (23;23') oder mit einer einzigen, als Langloch ausgebildeten Austrittsöffnung (36) versehen ist.

4. Vorrichtung nach Anspruch 3, **dadurch gekennzeichnet,** dass die mit den bogeninnenseitig angeordneten Austrittsöffnungen (23;23';36) versehene Sonde (20;20';35) an dem im Schlemmschen Kanal (15) angeordneten Ende verschlossen ist.

5. Vorrichtung nach den Ansprüchen 1 und 2, **dadurch gekennzeichnet,** dass lediglich an dem im Schlemmschen Kanal (15) angeordneten Ende der Sonde (26) eine in axialer Richtung derselben orientierte Austrittsöffnung (29) vorgesehen ist.

6. Vorrichtung nach Anspruch 1, **dadurch gekennzeichnet,** dass die an dem einen Ende jeweils mit den Austrittsöffnungen (23;23';29;36) versehenen Sonden (20;20';26;35) spiegelbildlich zueinander ausgebildet und an dem anderen Ende jeweils mit einem Stutzen (25;25') für einen kupplungsartigen Anschluss des Injektionsgerätes (30) versehen sind.

7. Vorrichtung nach den Ansprüchen 1 bis 6, **dadurch gekennzeichnet,** dass die Sonde (20;20';26;35) mit den daran angeordneten Austrittsöffnungen (23;23';29;36) zum einpressenden Injizieren eines hochviskosen Mediums in den Schlemmschen Kanal (15) ausgebildet ist, wobei die Flächen der einzelnen bei der hydraulischen Dehnung des Trabekulargewebes (15') traumatisch aufgeplatzten Öffnungen (15'') mit dem hochviskosen Medium benetzbar sind.

8. Vorrichtung nach den Ansprüchen 1 bis 7, **dadurch gekennzeichnet,** dass die Sonde (20;20';26;35) mit den daran angeordneten Austrittsöffnungen (23;23';29;36) zum einpressenden Injizieren einer chemisch modifizierten Hyaluronsäure ausgebildet ist, wobei die Hyaluronsäure ein in der Grössenordnung zwischen 3,2 x 10⁶ bis 5 x 10⁶ liegendes Molekulargewicht aufweist.

9. Vorrichtung nach den Ansprüchen 1 bis 7, **dadurch gekennzeichnet**, dass die Sonde (20;20';26;35) mit den daran angeordneten Austrittsöffnungen (23;23';29;36) zum einpressenden Injizieren eines Gemisches aus Hyaluronsäure mit Chondroitinsulfat beziehungeweise aus Hyaluronsäure mit Dextran ausgebildet ist.

10. Vorrichtung nach den Ansprüchen 1 bis 7, **dadurch gekennzeichnet,** dass die Sonde (20;20';26;35) mit den daran angeordneten Austrittsöffnungen (23;23';29;36) zum einpressenden Injizieren einer gelartigen Natrium-Hyaluronsäure ausgebildet ist.

## Claims

1. Device for reproducing the natural internal pressure in the eye of a living creature which is dependent on the supply and discharge of the aqueous humour, consisting of an injector and a tubular probe which is connected thereto and can be introduced through an incision into the substantially circular canal of Schlemm, characterized in that the probe (20; 20'; 26; 35) is arcuate in design and is provided on the arc interior (19; 19') and/or at the end arranged in the canal of Schlemm (15) with at least one respective outlet orifice (23; 23'; 29; 36) through which a medium can be injected by the injector (30) arranged at the other end of the probe (20; 20'; 26; 35), by means of which medium hydraulic expansion of the canal of Schlemm (15) and inevitable bursting of the trabecular meshwork (15') situated in front can be achieved and a connection between the canal of Schlemm (15) and the canalicular vein network of the trabecular meshwork (15') can be produced.

2. Device according to Claim 1, characterized in that the probe (20; 20'; 26; 35) produced from a tube is designed as an arcuate segment and has in each case a radius (R, R') substantially adapted to the canal of Schlemm (15) and measuring about 12 mm to 14 mm.

3. Device according to Claims 1 and 2, characterized in that the probe (20; 20'; 35) is provided on the arc interior (19; 19') with several mutually spaced outlet orifices (23; 23') or with a single outlet orifice (36) designed as a slot respectively.

4. Device according to Claim 3, characterized in that the probe (20; 20'; 35) provided with the outlet orifices (23; 23'; 36) arranged on the arc interior is closed at the end arranged in the canal of Schlemm (15).

5. Device according to Claims 1 and 2, characterized in that an outlet orifice (29) orientated in the axial direction of the probe (26) is provided only at the end of the probe (26) arranged in the canal of Schlemm (15).

6. Device according to Claim 1, characterized in that the probes (20; 20'; 26; 35) provided with the respective outlet orifices (23; 23'; 29; 36) at one end are designed in a mirror image to one another and are provided at the other end with a respective nozzle (25; 25') for a coupling-like connection of the injector (30).

7. Device according to Claims 1 to 6, characterized in that the probe (20; 20'; 26; 35) with the outlet orifices (23; 23'; 29; 36) arranged thereon is designed for the pressure injection of a high-viscosity medium into the canal of Schlemm (15), the faces of the individual orifices (15'') traumatically burst during hydraulic expansion of the trabecular meshwork (15'') being wettable by the high-viscosity medium.

8. Device according to Claims 1 to 7, characterized in that the probe (20; 20'; 26; 35) with the outlet orifices (23; 23'; 29; 36) arranged thereon is designed for the pressure injection of a chemically modified hyaluronic acid, the hyaluronic acid having a molecular weight between about 3.2 x 10⁶ and 5 x 10⁶.

9. Device according to Claims 1 to 7, characterized in that the probe (20; 20'; 26; 35) with the outlet orifices (23; 23'; 29; 36) arranged thereon is designed for the pressure injection of a mixture of hyaluronic acid with chondroitin sulphate or of hyaluronic acid with dextran.

10. Device according to Claims 1 to 7, characterized in that the probe (20; 20'; 26; 35) with the outlet orifices (23; 23'; 29; 36) arranged thereon is designed for the pressure injection of a gel-like sodium hyaluronic acid.

## Revendications

1. Dispositif permettant de restaurer la pression interne naturelle, fonction de l'écoulement d'entrée et de l'écoulement de sortie du liquide de la chambre oculaire, de l'oeil d'un être vivant, ledit dispositif étant constitué d'un appareil d'injection et d'une sonde tubulaire reliée à celui-ci et qui peut être introduite par une incision dans le canal de Schlemm de forme plus ou moins circulaire, caractérisé en ce que la sonde (20; 20'; 26; 35) est conformée en arc de cercle et est pourvue, respectivement, sur la face interne de l'arc (19; 19') et/ou sur la première extrémité agencée dans le canal de Schlemm (15), d'au moins une ouverture de sortie (23; 23'; 29; 36) qui permet, grâce à un appareil d'injection (30) agencé à l'autre extrémité de la sonde (20; 20'; 26; 35), d'injecter un milieu pour pouvoir effectuer une dilatation hydraulique du canal de Schlemm (15) ainsi qu'une fissuration forcée du tissu trabéculaire (15') disposé en amont et établir ainsi une liaison entre le canal de Schlemm (15) et le réseau veineux en forme de canal du tissu trabéculaire (15').

2. Dispositif selon la revendication 1, caractérisé en ce que la sonde (20; 20'; 26; 35) constituée d'un tube se présente sous la forme d'une partie segmentée en arc de cercle et présente, respectivement, un rayon (R, R') plus ou moins adapté au canal de Schlemm (15) et d'un ordre de grandeur de 12 à 14 mm.

3. Dispositif selon les revendications 1 et 2, caractérisé en ce que la sonde (20; 20'; 35) est pourvue, sur le côté interne du coude ou arc (19; 19'), respectivement de plusieurs ouvertures (23; 23') agencées à distance les unes des autres ou d'une seule ouverture (36) qui se présente sous la forme d'un orifice allongé.

4. Dispositif selon la revendication 3, caractérisé en ce que la sonde (20; 20'; 35) pourvue des ouvertures de sortie (23; 23'; 36) agencées côté interne du coude ou arc est fermée à l'extrémité agencée dans le canal de Schlemm (15).

5. Dispositif selon les revendications 1 et 2, caractérisé en ce qu'une ouverture de sortie (29) orientée dans la direction axiale de la sonde est ménagée uniquement à l'extrémité de la sonde (26) agencée dans le canal de Schlemm (15).

6. Dispositif selon la revendication 1, caractérisé en ce que les sondes (20; 20'; 26; 35) pourvues, à leur première extrémité, respectivement des ouvertures de sortie (23; 23'; 29; 36) ont une symétrie de miroir mutuelle et sont pourvues, à l'autre extrémité, respectivement d'un tube (25; 25') pour un raccordement par accouplement de l'appareil d'injection (30).

7. Dispositif selon les revendications 1 à 6, caractérisé en ce que la sonde (20; 20'; 26; 35) avec les ouvertures de sortie (23; 23'; 29; 36) qui y sont ménagées est conformée pour injecter par pression un milieu hautement visqueux dans le canal de Schlemm (15), les surfaces des ouvertures individuelles (15'') provoquées par traumatisme lors de la dilatation hydraulique du tissu trabéculaire pouvant être mouillées par le milieu hautement visqueux.

8. Dispositif selon les revendications 1 à 7, caractérisé en ce que la sonde (20; 20'; 26; 35) avec les ouvertures de sortie (23; 23'; 29; 36) qui y sont ménagées est conformée pour injecter par pression un acide hyaluronique chimiquement modifié, l'acide hyaluronique présentant un poids moléculaire d'un ordre de grandeur compris entre 3,2 x 10⁶ et 5 x 10⁶.

9. Dispositif selon les revendications 1 à 7, caractérisé en ce que la sonde (20; 20'; 26; 35) avec les ouvertures de sortie (23; 23'; 29; 36) qui y sont ménagées est conformée pour injecter par pression un mélange d'acide hyaluronique et de sulfate de chondroïtine, éventuellement d'acide hyaluronique et de dextranne.

10. Dispositif selon les revendications 1 à 7, caractérisé en ce que la sonde (20; 20'; 26; 35) avec les ouvertures de sortie (23; 23'; 29; 36) qui y sont ménagées est conformée pour injecter par pression un composé d'acide hyaluronique et de sodium se présentant sous la forme d'un gel.
